# EUROPEAN PATENT SPECIFICATION

(11) **EP 1 826 573 B1**
(45) Date of publication and mention of the grant of the patent: **11.04.2018**
(21) Application number: 06121345.0
(22) Date of filing: 27.09.2006
(51) Int. Cl.: G01N 35/02

(54) **Nucleic acid sample testing apparatus**
Gerät zum testen von Proben von Nukleinsäuren
Appareil pour tester des echantillions d'acide nucléique

(30) Priority: 04.10.2005 JP 2005291182; 04.08.2006 JP 2006213357
(43) Date of publication of application: 29.08.2007
(73) Proprietor: Canon Kabushiki Kaisha, Ohta-Ku, Tokyo 146-8501 (JP)
(72) Inventor: Sugiyama, Takahiro, Tokyo 146-8501 (JP); Araki, Yoshimasa, Tokyo 146-8501 (JP)
(74) Representative: TBK

(56) References cited:
- EP-A1- 1 431 390
- EP-A2- 0 977 039
- US-A1- 2002 028 489
- US-A1- 2004 181 050

## Description

### BACKGROUND OF THE INVENTION

### Field of the Invention

The present invention relates to a nucleic acid sample testing apparatus, and more particularly, to a nucleic acid sample testing apparatus, which is capable of processing a plurality of steps and equipped with a transport system for transporting a container.

### Description of the Related Art

In recent years, gene analyses using testing pieces, such as DNA microarrays and DNA chips, have been carried out. In any of those analyses, a DNA chip is brought into contact with a nucleic acid sample of a DNA or the like labeled with a fluorescent dye or the like under hybridization conditions. In this case, the DNA chip is provided as a detector on which a number of DNA probes are disposed and fixed as probe spots in a matrix form on the surface of a substrate which is composed of a slide glass, a silicon substrate, or the like. When both the detector (i.e., DNA chip) and the sample contain nucleic acids which can hybridize with each other, a labeled substance (i.e., labeled sample) can be fixed on the detector through a probe nucleic acid. The type of the hybridized nucleic acid can be specified by detecting where on the detector the labeled substance is present. A DNA microarray which utilizes such a hybridization reaction has been expected to be applied to medical diagnoses for specifying pathogens and gene diagnoses for examining constitutions or the like of patients.

In general, in any apparatus such as a nucleic acid sample testing apparatus, which uses a liquid for carrying out a reaction, the liquid such as a reagent or a sample should be dispensed into reaction containers using a dispensing device such as a pipette and the reaction containers each should be placed in a reaction area such as a temperature regulating section. Therefore, there is proposed an apparatus where a reagent and a reaction container are transferred to a dispensing device section or a reaction area by different transport systems (see, for example, Japanese Patent Application Laid-Open No. H09-096643).

In the nucleic acid sample testing apparatus, a processing including a plurality of different steps such as extraction, amplification, hybridization, and detection, is required to be carried out. In other words, those steps should be simultaneously carried out in parallel to allow an analysis to be quickly performed in large amounts. Furthermore, the processing includes two or more steps, so a size of the apparatus needs to be reduced by simplifying its configuration. In the apparatus disclosed in JP H09-096643 A, a reagent and a reaction container are arranged on different transport systems, and thus two transport systems are required. Furthermore, on the transport system for reaction, both a reaction section and a detecting section are arranged. In this case, however, there is a problem in that such a configuration of the apparatus makes it impossible for the steps of reaction and detection to be simultaneously

EP 0 977 039 A2 discloses an analysis apparatus for analyzing a biochemical analysis item being performed by a pipetting device which uses a repetitively used pipette nozzle, and an analysis apparatus for analyzing an immune analysis item being performed by a pipetting device which uses a disposable nozzle tip. A sample bottle containing a sample to be analyzed on both of a biochemical analysis item and an immune analysis item is sample-pipetted by the nozzle tip first, and then transported so as to be sample-pipetted by the pipette nozzle.

US 2002/028489 A discloses an automated analyzer for performing multiple diagnostic assays simultaneously includes multiple stations, or modules, in which discrete aspects of the assay are performed on fluid samples contained in reaction receptacles. The analyzer includes stations for automatically preparing a specimen sample, incubating the sample at prescribed temperatures for prescribed periods, preforming an analyte isolation procedure, and ascertaining the presence of a target analyte. An automated receptacle transporting system moves the reaction receptacles from one station to the next.; The analyzer further includes devices for carrying a plurality of specimen tubes and disposable pipette tips in a machine-accessible manner, a device for agitating containers of target capture reagents comprising suspensions of solid support material and for presenting the containers for machine access thereto, and a device for holding containers of reagents in a temperature controlled environment and presenting the containers for machine access thereto.

Therefore, it has been desired to provide a nucleic acid sample testing apparatus of a small size, which allows a reagent and a reaction container to be efficiently transferred to a desired position in each of the steps, and, after completion of the step, the processing is then allowed to proceed to a next processing step.

### SUMMARY OF THE INVENTION

In view of the circumstance of the above-mentioned conventional technology, it is an object of the present invention to provide a nucleic acid sample testing apparatus of a small size, which allows a reagent and a reaction container to be efficiently transferred to a desired position in each of the steps.

In order to achieve the above-mentioned object, a nucleic acid sample testing apparatus of the present invention according to claim 1 includes at least one reagent container; at least one reaction container; and a first transport unit for mounting the at least one reagent container and the at least one reaction container which is not connected with the at least one reagent container, and transporting the at least one reagent container and the at least one reaction container to a reaction area. Alternatively, the nucleic acid sample testing apparatus of the present invention according to claim 6 includes at least one reagent container; a reaction container holder for retaining the at least one reaction container, the reaction container holder not being connected with the at least one reagent container; and a first transport unit for mounting the at least one reagent container and the reaction container holder, and transporting the at least one reagent container and the at least one reaction container holder to a reaction area.

According to the present invention, it is possible to simplify the configuration of an apparatus because both a reagent and a reaction container are mounted on a single transport unit so that the single transport unit can transport them to a reaction area. In addition, a reagent container is provided independently of the reaction container, so only the reaction container can be
transported to a next processing step (i.e., detecting section) after completion of the reaction and only the reagent container can be returned to its original position and then recovered therefrom. Therefore, any undesired reagent cannot be introduced into the detecting section, so there is no fear of contaminating the detecting section. Meanwhile, because it is possible to transport only the reaction container to the detecting section, there is no need of unnecessary enlargement of the detecting section. In addition, a new examination can be initiated by mounting a subsequent sample after the reagent is recovered, so it is possible to initiate a parallel processing efficiently.

Further features of the present invention will become apparent from the following description of exemplary embodiments with reference to the attached drawings.

### BRIEF DESCRIPTION OF THE DRAWINGS

FIG. 1 is a front view of a nucleic acid sample testing apparatus according to a first embodiment of the present invention.
FIG. 2 is a top view of the nucleic acid sample testing apparatus according to the first embodiment of the present invention.
FIG. 3 is a right side view of the nucleic acid sample testing apparatus according to the first embodiment of the present invention.
FIG. 4 is a cross-sectional view of the nucleic acid sample testing apparatus taken along the dashed line 4-4 of FIG. 2.
FIG. 5 is a cross-sectional view of the nucleic acid sample testing apparatus taken along the dashed line 5-5 of FIG. 1.
FIG. 6 is a cross-sectional view of the nucleic acid sample testing apparatus taken along the dashed line 6-6 of FIG. 5.
FIG. 7 is a front view of part of the nucleic acid sample testing apparatus, for explaining movement of the apparatus when a hybridization process is carried out in the first embodiment of the present invention.
FIG. 8 is a front view of part of the nucleic acid sample testing apparatus, for explaining movement of the apparatus when a hybridization process is carried out in the first embodiment of the present invention.
FIG. 9 is a front view of part of the nucleic acid sample testing apparatus, for explaining movement of the apparatus when a hybridization process is carried out in the first embodiment of the present invention.
FIG. 10 is a diagram that illustrates a state in which a spring is attached on a pressure-contacting block to be used in the steps shown in FIGS. 7 to 9.
FIG. 11 is a front view of part of a nucleic acid sample testing apparatus according to a second embodiment of the present invention.

### DESCRIPTION OF THE EMBODIMENTS

Hereinafter, embodiments of the present invention will be described with reference to the attached drawings.

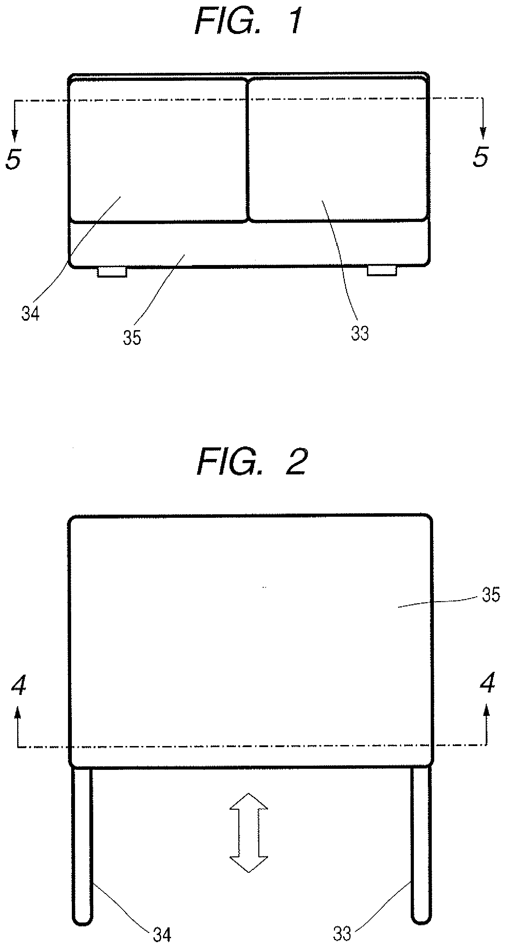

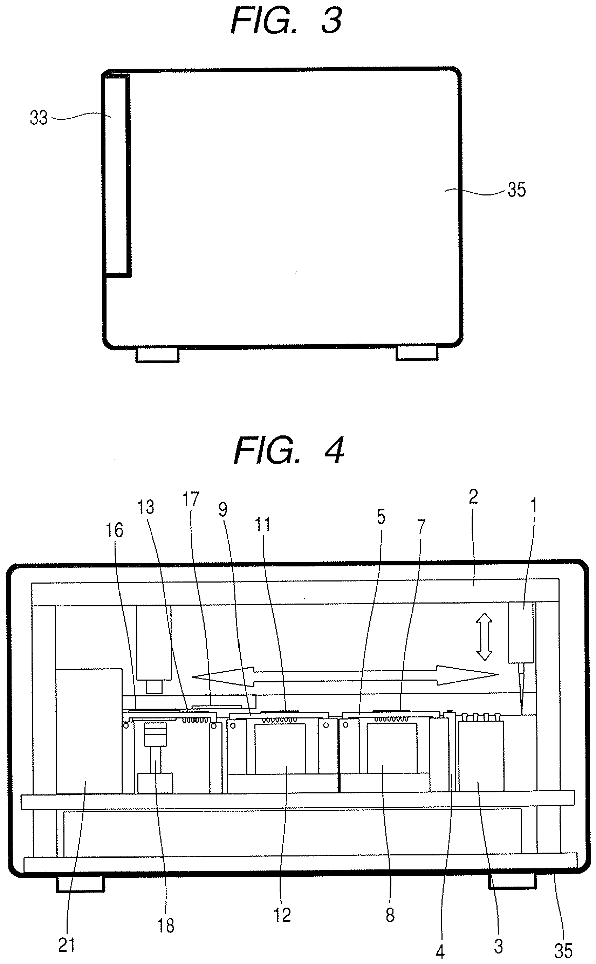

FIG. 1 is a front view of a nucleic acid sample testing apparatus according to a first embodiment of the present invention. A right door 33 and a left door 34 provided on the front of the nucleic acid sample testing apparatus 35 are opened and a well or the like is then placed on an installation site in the apparatus. FIG. 2 is a top view of the nucleic acid sample testing apparatus according to the first embodiment of the present invention. In the figure, the right and left doors 33 and 34 are being opened. FIG. 3 is a right side view of the nucleic acid sample testing apparatus according to the first embodiment of the present invention. FIG. 4 is a cross-sectional view of the nucleic acid sample testing apparatus taken along the dashed line 4-4 of FIG. 2.

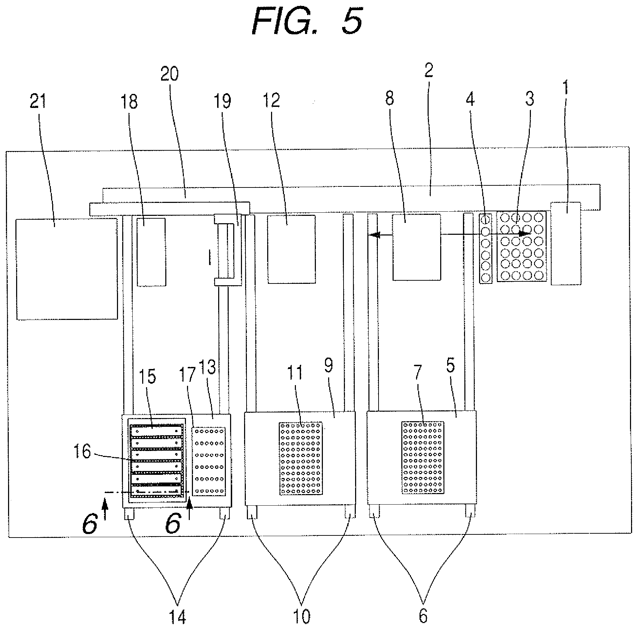

FIG. 5 is a cross-sectional view of the nucleic acid sample testing apparatus taken along the dashed line 5-5 of FIG. 1.

Referring to FIGS. 4 and 5, a pipette unit 1 for handling a liquid such as a sample or a reagent is arranged such that the pipette unit 1 is connected to a pipette guide 2 for transferring the pipette unit 1. In addition, within a movable range of the pipette unit 1, a pipette chip storage space 3 for placing an unused pipette chip thereon and a sample storage space 4 are arranged.

A transport carrier 5 and a transport guide 6, which are provided for a first step, are arranged in the direction along which the pipette unit 1 moves and adjacent to the sample storage space 4. The transport carrier 5 is able to move along the transport guide 6 in the horizontal direction perpendicular to the direction along which the pipette unit 1 moves. On the transport carrier 5, a reagent container 7 is placed. In addition, within the range of movement of the transport carrier 5 and also within the movable range of the pipette unit 1, a processing section 8 is arranged.

In the same manner as in the first step, a transport carrier 9 and a transport guide 10, which are provided for a second step, are arranged in the direction along which the pipette unit 1 moves and adjacent to an area where the above-mentioned transport carrier 5 is allowed to move. The transport carrier 9 is able to move along the transport guide 10 in the horizontal direction perpendicular to the direction along which the pipette unit 1 moves. On the transport carrier 9, a reagent container 11 is placed. In addition, within the range of movement of the transport carrier 9 and also within the movable range of the pipette unit 1, a processing section 12 is arranged.

Furthermore, a transport carrier 13 and a transport guide 14, which are provided for a third step, are arranged in the direction along which the pipette unit 1 moves and adjacent to an area where the above transport carrier 9 is allowed to move. The transport carrier 13 is able to move along the transport guide 14 in the horizontal direction perpendicular to the direction along which the pipette unit 1 moves. On the transport carrier 13, a tray 16 on which a plurality of DNA microarrays 15 each serving as a reaction container are mounted, and a reagent container 17 are arranged without any connection therebetween. In addition, a processing section 18 is arranged within the range of movement of the transport carrier 13 and also within the movable range of the pipette unit 1. It should be noted that, in this embodiment, a reagent container 17 which is capable of housing a plurality of reagents, and one tray 16 on which a plurality of DNA microarrays 15 are mounted are arranged without any connection therebetween on a transport carrier 13. However, the numbers of the reagent containers 17 and the DNA microarrays 15 to be mounted on the transport carrier 13 are arbitrary, and thus the numbers thereof are not limited to the numbers represented in the drawings.

Furthermore, above the range of movement of the transport carrier 13, a transport unit 19 is arranged while being connected to a transport guide 20. The transport unit 19 can move in the same direction as that of movement of the pipette unit 1 along the transport guide 20. In addition, a detecting section 21 is arranged on the movable range of the transport unit 19.

In the above-mentioned configuration of the apparatus, when a sample is placed on the sample storage space 4, a pipette chip is placed in the pipette chip storage space 3, and a reagent container 7 containing a reagent is placed on the transport carrier 5, processing of the first step is initiated.

However, the conditions for initiating the processing are not limited thereto. The processing may be designed such that it can be initiated only when the reagent containers 11 and 17 each containing a reagent and the tray 16 mounted with the DNA microarrays 15 are placed on each of the transport carriers 9 and 13.

First, for carrying out the processing in the first step, the transport carrier 5 is moved to the processing section 8. Next, the pipette unit 1 is prepared such that an unused pipette chip is attached on the pipette unit 1 in the pipette chip storage space 3 and then used to suck the sample in the sample storage space 4. Subsequently, the pipette unit 1 is moved to the position of the processing section 8, where the pipette unit 1 discharges the sample into the reagent container 7. After that, a predetermined processing is carried out in the processing section 8, thereby completing the processing in the first step. Here, the phrase "the processing in the first step" refers to, for example, the processing for extraction and purification, where the first step includes mixing and stirring of the reagent. After completion of the processing for extraction and purification, a DNA extracted from the sample is set to a place in the reagent container 7.

Next, for carrying out the processing in the second step, the transport carrier 9 is moved to the processing section 12. On the other hand, the pipette unit 1 sucks the product of the first-step processing from the reagent container 7 using a new pipette chip and then discharges the product into the reagent container 11. Subsequently, a predetermined processing is carried out in the reagent container 11, thereby completing the processing in the second step. Here, the phrase "the processing in the second step" refers to, for example, an amplification processing, which includes steps of mixing and stirring of the reagent, and regulating temperature. After completion of the amplification processing, an amplified DNA is set to a place in the reagent container 11.

Next, for carrying out the processing in the third step, the transport carrier 13 is moved to the processing section 18. On the other hand, the pipette unit 1 sucks the product of the second-step processing from the reagent container 11 using a new pipette chip and then discharges the product into the regent container 17. Subsequently, in the reagent container 17, the product is mixed and stirred with the reagent, thereby preparing a mixture thereof. Then, the mixture is set in a place on a DNA microarray 15, while the processing section 18 regulates temperature, to carry out the processing in the third step. Here, the phrase "the processing in the third step" refers to, for example, hybridization. The DNA microarray 15 may be designed to reserve the mixture on a DNA probe to allow a hybridization reaction. For instance, the DNA microarray 15 may have a cover thereover, or may be designed to have a cartridge structure in which an inlet, a flow channel, a chamber, and an outlet for the liquid are formed.

When the third step is completed, the transport unit 19 is allowed to move the tray 16 equipped with the DNA microarrays 15 to the detecting section 21 along the transport guide 20, thereby detecting the results of the reaction. Subsequently, the transport carrier 13 evacuates the reagent container 17 to a position for recovering a reagent container (i.e., a position far from the processing section 18 as shown in FIG. 5), thereby allowing the reagent container 17 to be recovered. If required, during the detection at the detecting section 21, both DNA microarrays and reagent containers for a subsequent test are set in respective places, thereby allowing parallel processing.

In this embodiment, the pipette chips used are discarded by a method (not shown) after completion of the processing in all steps. In addition, the reagent containers 7, 11, and 17 each are also used as a container for mixing or reaction of the reagent. The reagent containers can be recovered or discarded after completion of the respective steps by any means (not shown).

Furthermore, in the above embodiment, the apparatus has a configuration in which all reagents are mounted on the transport carrier while the reagents are each previously placed in the reagent container. Alternatively, the apparatus may not employ such the configuration and may be configured so that the reagent is previously retained in the apparatus and then transferred to the reagent container by a pipette or another means.

Furthermore, the sample storage space 4 may be configured such that it can be set on the transport carrier and then used.

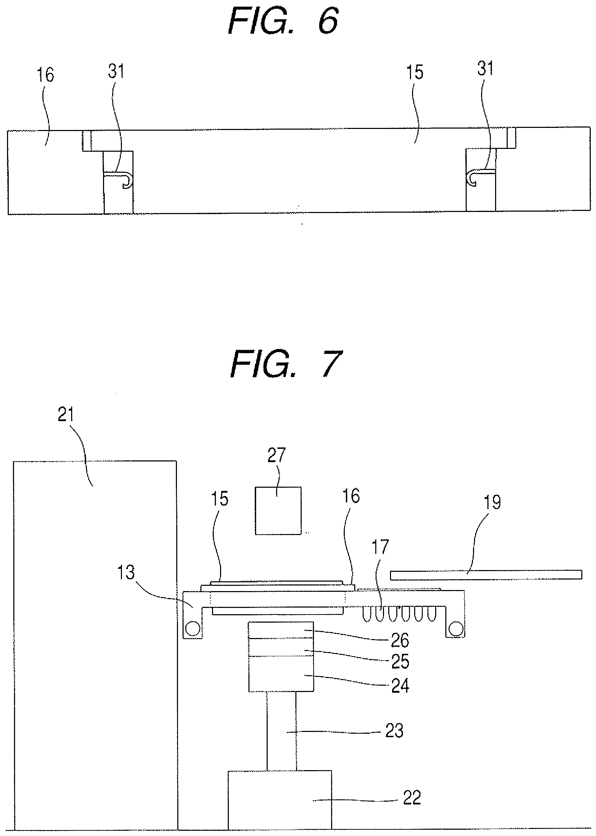

FIG. 6 is a cross-sectional view taken along the dashed line 6-6 of FIG. 5, showing a detail of the configuration of the tray 16 on which the DNA microarray 15 is mounted.

In the tray 16, stepped holes are formed, where the respective DNA microarrays 15 can be dropped in, and the DNA microarrays 15 are provided in the respective holes. Holding members 31 are formed on the inner side of each of the holes. When the DNA microarray 15 is placed in the hole, the holding members 31 press/hold the DNA microarray 15 so that the DNA microarray 15 can be held in the tray without coming out. The way for holding the DNA microarray 15 is not limited to such the configuration, and any of structures having at least an ability of preventing the DNA microarray from coming out of the tray 16 may be employed. Note that, such the tray 16 is mounted on the transport carrier 13 in a manner that the tray 16 is placed in a hole passing through the transport carrier 13 (see FIGS. 7 to 10).

FIGS. 7 to 10 sequentially show a series of movements of the apparatus when the hybridization described above is carried out. Note that, each of those figures is a front view of part of the nucleic acid sample testing apparatus of this embodiment.

FIG. 7 shows the configuration of the apparatus immediately before the hybridization. The tray 16 provided with a plurality of DNA microarrays 15 and the reagent container 17 containing a plurality of reagents are placed on the transport carrier 13, while they are not connected with each other. The transport unit 19 is arranged above the transport carrier 13 moved to a predetermined reaction position (i.e., the processing section 18 shown in FIG. 5). On the same position, a motor 22, a shaft for vertical movement 23, an ascending/descending table 24, a Pertier element 25, and a heat block 26 are arranged below the DNA microarray 15. Furthermore, a press-contacting block 27 is arranged above the DNA microarray 15. In addition, the detecting section 21 is arranged on the left side of the transport carrier 13.

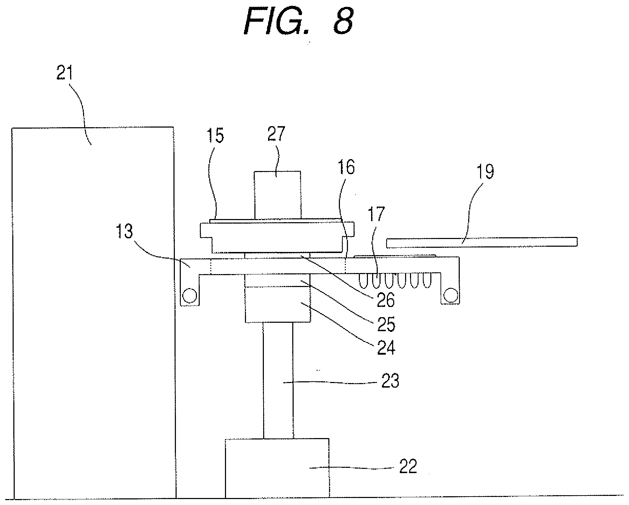

FIG. 8 shows the configuration of the apparatus under hybridization. For adjusting the temperature of the DNA microarray 15, the motor 22 is actuated to move the ascending/descending table 24 upward along the shaft for vertical movement 23. As a result, the bottom surface of the DNA microarray 15 is brought into contact with the heat block 26. After that, the ascending/descending table 24 moves upward until the upper surface of the DNA microarray 15 is brought into contact with the press-contacting block 27 fixed on the body (not shown). Consequently, the bottom surface of the DNA microarray 15 is brought into close contact with the heat block 26. At this time, when the DNA microarray 15 is lifted up, the tray 16 is moved upward together with the DNA microarray 15 and separated from the transport carrier 13 due to the configuration of the apparatus as described in FIG. 6. Next, the control of the temperature of the DNA microarray 15 is initiated when a control unit (not shown) initiates the temperature control on the Pertier element 25. Thus, by setting a sample and a regent for hybridization in a place on the DNA microarray 15, a hybridization reaction can be carried out.

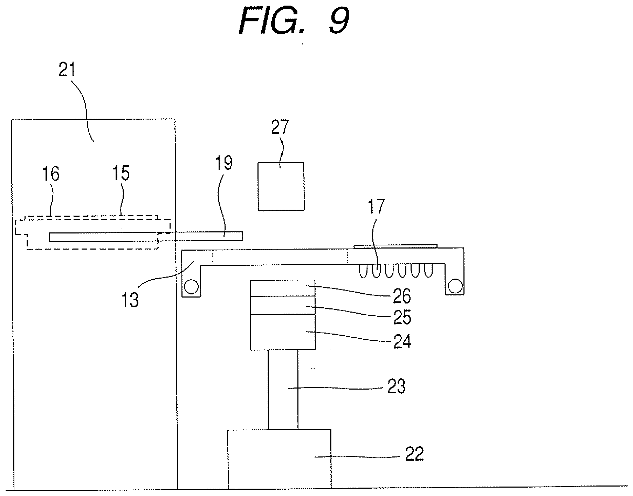

FIG. 9 shows the configuration of the apparatus after completion of the hybridization reaction. In this state, the transport unit 19 has transported the tray 16 equipped with the DNA microarray 15 to the detecting section 21. From the state shown in FIG. 8, the transport unit 19 is moved downward to a predetermined position. After the movement, the motor 22 is actuated to move the heat block 26 downward along the shaft for vertical movement 23, thereby allowing the tray 16 equipped with the DNA microarray 15 to be set in a place on the transport unit 19. Subsequently, as shown in Fig. 9, when the transport unit 19 moves the tray 16 to the detecting section 21 and then set it in a place thereon to detect the result of hybridization. After that, the transport unit 19 is evacuated to the position shown in FIG. 7 and the transport carrier 13 is moved to the position far from the processing section 18 as shown in FIG. 5, thereby recovering the reagent container 17. In this examination, if required, another tray 16 equipped with the DNA microarray 15 for a subsequent examination and an additional reagent container 17 are set in a place on the transport carrier 13, thereby allowing parallel processing. In other words, in this embodiment, only the tray 16 equipped with the DNA microarray 15 is transported to the detecting section 21 during the detection after the reaction, so the transport carrier 13 is allowed to be equipped with a next sample to initiate an additional experiment. Thus, it is possible to carry out parallel processing efficiently. Furthermore, an undesired reagent is not introduced into the detecting section 21, so there is no fear of contaminating the detecting section 21. Furthermore, only the tray 16 equipped with the DNA microarray 15 is transported to the detecting section 21, so there is no need of unnecessary enlargement of the detecting section 21.

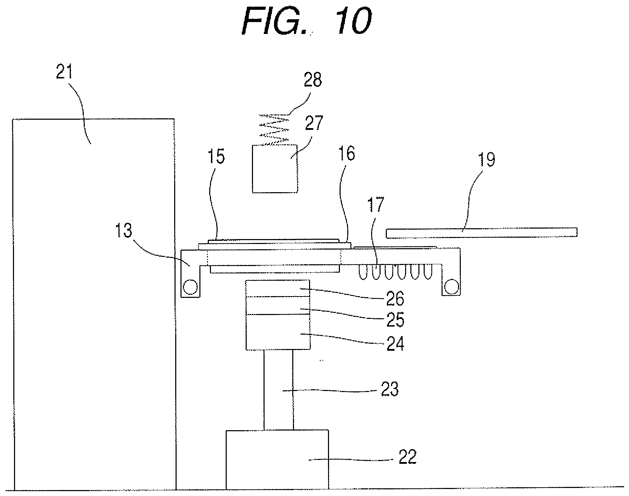

Note that, it is preferable to provide the press-contacting block 27 with a spring, when the press-contacting block 27 is used in the hybridization as shown in FIGS. 7 to 9. FIG. 10 shows an example of such a configuration. When hybridization is carried out, it is only necessary that the bottom surface of the DNA microarray 15 is brought into close contact with the heat block 26. It is possible to obtain a more stable press-contacting force by providing the press-contacting block 27 with the spring 28 as shown in FIG. 10. Consequently, a more stable fitness of the DNA microarray 15 with the heat block 26 can be obtained.

In the above embodiment, during the hybridization, both the DNA microarray 15 and the tray 16 are separated from the transport carrier 13 as shown in FIG. 8. Thus, for example, as far as the transport carrier 13 is within the range where the transport carrier 13 does not interfere with the heat block 26, the reagent container 17 may be moved if required. For instance, the reagent container 17 may be shifted from its position and then subjected to pipetting.

Furthermore, in the above embodiment, a temperature control mechanism is moved up and down to serve both the function of allowing the DNA microarray as a reaction container to come into close contact with the heat block 26 and the function of separating the tray 16 from the transport carrier 13. Alternatively, the temperature control mechanism and an ascending/descending mechanism may be configured separately.

Next, the second embodiment of the present invention will be described.

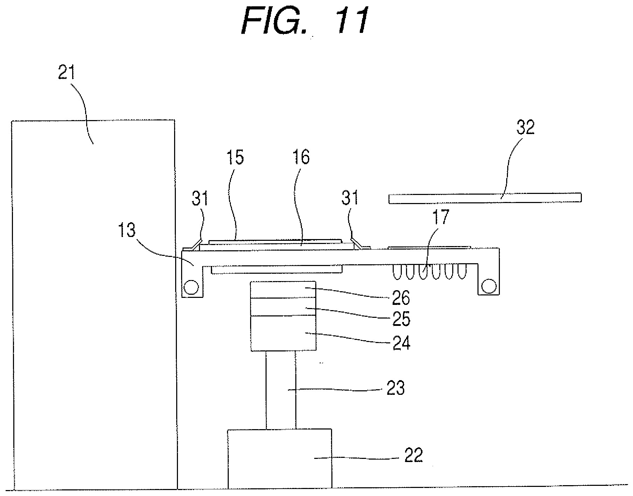

Here, another example of a mechanism around the transport carrier 13 for carrying out the third step is described. FIG. 11 is a front view of part of a nucleic acid sample testing apparatus of the second embodiment. Here, in this figure, the same mechanisms and parts as those of the first embodiment are denoted by the same reference numerals.

As shown in FIG. 11, on a transport carrier 13 for carrying out the third step, a tray 16 on which a DNA microarray 15 is arranged and reagent containers 17 are arranged. On the upper surface of the transport carrier 13, a tray-fixing mechanism 31 for fixing the tray 16 is provided. A transport unit 32, which can move the tray 16 upward and downward, is arranged above the transport carrier 13. In addition, below the DNA microarray 15, a motor 22, a shaft for vertical movement 23, an ascending/descending table 24, a Pertier element 25, and a heat block 26 are disposed. On the left side of the transport carrier 13, a detecting section 21 is arranged.

In the above configuration of the apparatus, when the transport carrier 13 is moved to a predetermined reaction position, then a reaction is initiated. First, the tray fixing mechanism 31 presses/holds the tray 16 from above against the transport carrier 13 so that the tray 16 does not move upward and downward. Then, the motor 22 is actuated to move the heat block 26 upward along the shaft for vertical movement 23, so the bottom surface of the DNA microarray 15 is brought into contact with the heat block 26. At this time, the tray fixing mechanism 31 presses/holds the tray 16, so the fitness between the DNA microarray 15 and the heat block 26 can be retained. Subsequently, a control unit (not shown) initiates the temperature control on the Pertier element 25, thereby initiating the temperature control of the DNA microarray 15. Hybridization reaction can be carried out after a sample and a reagent for hybridization are placed on the DNA microarray 15. After completion of the hybridization reaction, the motor 22 is actuated to move the heat block 26 downward. On the other hand, the tray fixing mechanism 31 releases the fixation of the tray 16. After that, the transport unit 32 brings up the tray 16 from the transport carrier 13, followed by transport of the tray 16 to the detecting section 21. The tray 16 may be transported such that it is mounted on the top side of the transport unit 32, or the tray 16 may be transported by sucking it by air or by means of a magnet such that the tray is arranged on the bottom side of the transport unit 32.

In each of the above first and second embodiments, the DNA microarray 15 is placed on the tray 16 and then mounted on the transport carrier 13. Alternatively, it should be noted that the DNA microarray 15 may be directly mounted on the transport carrier 13. In this case, the transport unit 19 or 32 transports the DNA microarrays 15 one by one to the detecting section 21, or a plurality of the DNA microarrays 15 may be simultaneously arranged on the transport unit 19 or 32.

While the present invention has been described with reference to exemplary embodiments, it is to be understood that the invention is not limited to the disclosed exemplary embodiments.

A nucleic acid sample testing apparatus includes a transport carrier, which can move along a transport guide in a horizontal direction perpendicular to a direction of movement of a pipette unit. On the transport carrier, a tray equipped with a DNA microarray as a reaction container and a reagent container are arranged without being connected with each other. In addition, within a range of movement of the transport carrier and also within a movable range of the pipette unit, a processing section for carrying out a hybridization reaction is arranged. Above the range of movement of the transport carrier, a transport unit is arranged while being connected with a transport guide. The transport unit can move in the same direction as the moving direction of the pipette unit along the transport guide. In addition, a detecting section is arranged on the movable range of the transport unit.

## Claims

1. A nucleic acid sample testing apparatus, comprising:
at least one reagent container (7, 11, 17);
at least one reaction container (15);
a pipette unit (1); and
a transport carrier (5, 9, 13) for mounting the at least one reagent container (7, 11, 17) and the at least one reaction container (15) which is not connected with the at least one reagent container (7, 11, 17), and transporting the at least one reagent container (7, 11, 17) and the at least one reaction container (15) to a processing section (8, 12, 18),
wherein the transport carrier (5, 9, 13) is able to move along a transport guide (6) in the horizontal direction perpendicular to the direction along which the pipette unit (1) moves.

2. A nucleic acid sample testing apparatus according to claim 1, further comprising:
a transport unit (19; 32) for transporting the at least one reaction container (15) transported to the processing section (8, 12, 18) by the transport carrier (5, 9, 13) from a place on the transport carrier (5, 9, 13) to a next section (4, 8, 12, 18, 21) after completion of a reaction in the processing section (8, 12, 18).

3. A nucleic acid sample testing apparatus according to claim 2, wherein
the at least one reaction container (15) is transported one by one to the next section (4, 8, 12, 18, 21) from the place on the transport carrier (5,9, 13).

4. A nucleic acid sample testing apparatus according to claim 2, wherein
the at least one reaction container (15) is transported in a plural number to the next section (4, 8, 12, 18, 21) from the place on the transport carrier (5, 9, 13).

5. A nucleic acid sample testing apparatus according to claim 2, wherein the transport carrier (5, 9, 13) transports only the reagent container (7, 11, 17) to its original position after the transport unit (19; 32) transfers the reaction container (15) to the next section (4, 8, 12, 18, 21).

6. A nucleic acid sample testing apparatus, comprising:
at least one reagent container (7, 11, 17);
a reaction container holder (16) for retaining at least one reaction container (15), the reaction container holder (16) not being connected with the at least one reagent container (7, 11, 17);
a pipette unit (1); and
a transport carrier (5, 9, 13) for mounting the at least one reagent container (7, 11, 17) and the reaction container holder (16), and transporting the at least one reagent container (7, 11, 17) and the reaction container holder (16) to a processing section (8, 12, 18),
wherein the transport carrier (5, 9, 13) is able to move along a transport guide (6) in the horizontal direction perpendicular to the direction along which the pipette unit (1) moves.

7. A nucleic acid sample testing apparatus according to claim 6, further comprising:
a transport unit (19; 32) for transporting the reaction container holder (16) transported to the processing section (8, 12, 18) by the transport carrier (5, 9, 13) from a place on the transport carrier (5, 9, 13) to a next section (4, 8, 12, 18, 21) after completion of a reaction in the processing section (8, 12, 18).

8. A nucleic acid sample testing apparatus according to claim 7, wherein
the at least one reaction container (15) in the reaction container holder (16) is transported one by one to the next section (4, 8, 12, 18, 21) from the place on the transport carrier (5, 9, 13).

9. A nucleic acid sample testing apparatus according to claim 7, wherein
the at least one reaction container (15) in the reaction container holder (16) is transported in a plural number to the next section (4, 8, 12, 18, 21) from the place on the transport carrier (5, 9, 13).

10. A nucleic acid sample testing apparatus according to claim 7, wherein
the transport carrier (5, 9, 13) transports only the at least one reagent container (7, 11, 17) to its original position after the transport unit (19; 32) transports the reaction container holder (16) to the next section (4, 8, 12, 18, 21).

11. A nucleic acid sample testing apparatus according to any one of claims 1 to 10, wherein
the reaction container (15) includes a DNA microarray (15).

## Patentansprüche

1. Vorrichtung zum Testen von Nukleinsäureproben, die Folgendes aufweist:
zumindest einen Reagenzbehälter (7, 11, 17);
zumindest einen Reaktionsbehälter (15);
eine Pipetteneinheit (1); und
einen Transportträger (5, 9, 13) zum Befestigen des zumindest einen Reagenzbehälters (7, 11, 17) und des zumindest einen Reaktionsbehälters (15), der nicht mit dem zumindest einen Reagenzbehälter (7, 11, 17) verbunden ist, und zum Transportieren des zumindest einen Reagenzbehälters (7, 11, 17) und des zumindest einen Reaktionsbehälters (15) zu einem Verarbeitungsabschnitt (8, 12, 18),
wobei der Transportträger (5, 9, 13) in der Lage ist, sich entlang einer Transportführung (6) in der horizontalen Richtung senkrecht zu der Richtung zu bewegen, entlang der sich die Pipetteneinheit (1) bewegt.

2. Vorrichtung zum Testen von Nukleinsäureproben nach Anspruch 1, ferner mit:
einer Transporteinheit (19; 32) zum Transportieren des zumindest einen Reaktionsbehälters (15), der durch den Transportträger (5, 9, 13) zu dem Verarbeitungsabschnitt (8, 12, 18) transportiert ist, von einer Stelle auf dem Transportträger (5, 9, 13) zu einem nächsten Abschnitt (4, 8, 12, 18, 21) nach einer Beendigung einer Reaktion in dem Verarbeitungsabschnitt (8, 12, 18).

3. Vorrichtung zum Testen von Nukleinsäureproben nach Anspruch 2, wobei
der zumindest eine Reaktionsbehälter (15) einer nach dem anderen von der Stelle auf dem Transportträger (5, 9, 13) aus zu dem nächsten Abschnitt (4, 8, 12, 18, 21) transportiert wird.

4. Vorrichtung zum Testen von Nukleinsäureproben nach Anspruch 2, wobei
der zumindest eine Reaktionsbehälter (15) in einer Vielzahl von der Stelle auf dem Transportträger (5, 9, 13) aus zu dem nächsten Abschnitt (4, 8, 12, 18, 21) transportiert wird.

5. Vorrichtung zum Testen von Nukleinsäureproben nach Anspruch 2, wobei der Transportträger (5, 9, 13) lediglich den Reagenzbehälter (7, 11, 17) zu dessen Ursprungsposition hin transportiert, nachdem die Transporteinheit (19; 32) den Reaktionsbehälter (15) zu dem nächsten Abschnitt (4, 8, 12, 18, 21) transportiert.

6. Vorrichtung zum Testen von Nukleinsäureproben, die Folgendes aufweist:
zumindest einen Reagenzbehälter (7, 11, 17);
einen Reaktionsbehälterhalter (16) zum Halten von zumindest einem Reaktionsbehälter (15), wobei der Reaktionsbehälterhalter (16) nicht mit dem zumindest einen Reagenzbehälter (7, 11, 17) verbunden ist;
eine Pipetteneinheit (1); und
einen Transportträger (5, 9, 13) zum Befestigen von zumindest einem Reagenzbehälter (7, 11, 17) und dem Reaktionsbehälterhalter (16) und zum Transportieren des zumindest einen Reagenzbehälters (7, 11, 17) und des Reaktionsbehälterhalters (16) zu einem Verarbeitungsabschnitt (8, 12, 18),
wobei der Transportträger (5, 9, 13) in der Lage ist, sich entlang einer Transportführung (6) in der horizontalen Richtung senkrecht zu der Richtung zu bewegen, entlang der sich die Pipetteneinheit (1) bewegt.

7. Vorrichtung zum Testen von Nukleinsäureproben nach Anspruch 6, ferner mit:
einer Transporteinheit (19; 32) zum Transportieren des Reaktionsbehälterhalters (16), der durch den Transportträger (5, 9, 13) zu dem Verarbeitungsabschnitt (8, 12, 18) transportiert wird, von einer Stelle auf dem Transportträger (5, 9, 13) zu einem nächsten Abschnitt (4, 8, 12, 18, 21) nach einer Beendigung einer Reaktion in dem Verarbeitungsabschnitt (8, 12, 18).

8. Vorrichtung zum Testen von Nukleinsäureproben nach Anspruch 7, wobei
der zumindest eine Reaktionsbehälter (15) in dem Reaktionsbehälterhalter (16) einer nach dem anderen von der Stelle auf dem Transportträger (5, 9, 13) aus zu dem nächsten Abschnitt (4, 8, 12, 18, 21) transportiert wird.

9. Vorrichtung zum Testen von Nukleinsäureproben nach Anspruch 7, wobei
der zumindest eine Reaktionsbehälter (15) in dem Reaktionsbehälterhalter (16) in einer Vielzahl von der Stelle auf dem Transportträger (5, 9, 13) aus zu dem nächsten Abschnitt (4, 8, 12, 18, 21) transportiert wird.

10. Vorrichtung zum Testen von Nukleinsäureproben nach Anspruch 7, wobei
der Transportträger (5, 9, 13) lediglich den zumindest einen Reagenzbehälter (7, 11, 17) zu dessen Ursprungsposition hin transportiert, nachdem die Transporteinheit (19; 32) den Reaktionsbehälterhalter (16) zu dem nächsten Abschnitt (4, 8, 12, 18, 21) transportiert.

11. Vorrichtung zum Testen von Nukleinsäureproben nach einem von Ansprüchen 1 bis 10, wobei
der Reaktionsbehälter (15) ein DNA-Mikroarray (15) aufweist.

## Revendications

1. Appareil pour tester des échantillons d'acide nucléique, comprenant :
au moins un contenant de réactif (7, 11, 17) ;
au moins un contenant de réaction (15) ;
une unité de pipette (1) ; et
un support de transport (5, 9, 13) destiné à porter ledit au moins un contenant de réactif (7, 11, 17) et ledit au moins un contenant de réaction (15) qui n'est pas relié audit au moins un contenant de réactif (7, 11, 17), et transporter ledit au moins un contenant de réactif (7, 11, 17) et ledit au moins un contenant de réaction (15) vers une section de traitement (8, 12, 18),
dans lequel le support de transport (5, 9, 13) est apte à se déplacer le long d'un guide de transport (6) dans la direction horizontale perpendiculaire à la direction selon laquelle l'unité de pipette (1) se déplace.

2. Appareil pour tester des échantillons d'acide nucléique selon la revendication 1, comprenant en outre une unité de transport (19 ; 32) destinée à transporter ledit au moins un contenant de réaction (15) transporté vers la section de traitement (8, 12, 18) par le support de transport (5, 9, 13) depuis un emplacement sur le support de transport (5, 9, 13) vers une section suivante (4, 8, 12, 18, 21) après achèvement d'une réaction dans la section de traitement (8, 12, 18).

3. Appareil pour tester des échantillons d'acide nucléique selon la revendication 2, dans lequel ledit au moins un contenant de réaction (15) est transporté un à un vers la section suivante (4, 8, 12, 18, 21) depuis l'emplacement sur le support de transport (5, 9, 13).

4. Appareil pour tester des échantillons d'acide nucléique selon la revendication 2, dans lequel ledit au moins un contenant de réaction (15) est transporté par grand nombre vers la section suivante (4, 8, 12, 18, 21) depuis l'emplacement sur le support de transport (5, 9, 13).

5. Appareil pour tester des échantillons d'acide nucléique selon la revendication 2, dans lequel le support de transport (5, 9, 13) transporte uniquement le contenant de réactif (7, 11, 17) vers sa position d'origine après que l'unité de transport (19 ; 32) transfère le contenant de réaction (15) vers la section suivante (4, 8, 12, 18, 21).

6. Appareil pour tester des échantillons d'acide nucléique, comprenant :
au moins un contenant de réactif (7, 11, 17) ;
un porte-contenant de réaction (16) destiné à contenir au moins un contenant de réaction (15), le porte-contenant de réaction (16) n'étant pas relié audit au moins un contenant de réactif (7, 11, 17) ;
une unité de pipette (1) ; et
un support de transport (5, 9, 13) destiné à porter ledit au moins un contenant de réactif (7, 11, 17) et le porte-contenant de réaction (16), et transporter ledit au moins un contenant de réactif (7, 11, 17) et le porte-contenant de réaction (16) vers une section de traitement (8, 12, 18),
dans lequel le support de transport (5, 9, 13) est apte à se déplacer le long d'un guide de transport (6) dans la direction horizontale perpendiculaire à la direction selon laquelle l'unité de pipette (1) se déplace.

7. Appareil pour tester des échantillons d'acide nucléique selon la revendication 6, comprenant en outre une unité de transport (19 ; 32) destinée à transporter le porte-contenant de réaction (16) transporté vers la section de traitement (8, 12, 18) par le support de transport (5, 9, 13) depuis un emplacement sur le support de transport (5, 9, 13) vers une section suivante (4, 8, 12, 18, 21) après achèvement d'une réaction dans la section de traitement (8, 12, 18).

8. Appareil pour tester des échantillons d'acide nucléique selon la revendication 7, dans lequel ledit au moins un contenant de réaction (15) dans le porte-contenant de réaction (16) est transporté un à un vers la section suivante (4, 8, 12, 18, 21) depuis l'emplacement sur le support de transport (5, 9, 13).

9. Appareil pour tester des échantillons d'acide nucléique selon la revendication 7, dans lequel ledit au moins un contenant de réaction (15) dans le porte-contenant de réaction (16) est transporté par grand nombre vers la section suivante (4, 8, 12, 18, 21) depuis l'emplacement sur le support de transport (5, 9, 13).

10. Appareil pour tester des échantillons d'acide nucléique selon la revendication 7, dans lequel le support de transport (5, 9, 13) transporte uniquement ledit au moins un contenant de réactif (7, 11, 17) vers sa position d'origine après que l'unité de transport (19 ; 32) transporte le porte-contenant de réaction (16) vers la section suivante (4, 8, 12, 18, 21).

11. Appareil pour tester des échantillons d'acide nucléique selon l'une quelconque des revendications 1 à 10, dans lequel le contenant de réaction (15) comprend un microréseau d' ADN (15).
